# EUROPEAN PATENT APPLICATION

(11) **EP 4 510 039 A1**
(43) Date of publication of application: **19.02.2025**
(21) Application number: 24172918.5
(22) Date of filing: 29.04.2024
(51) Int. Cl.: G06N 3/045, G06N 3/0895, G06F 16/901, G06Q 10/107, G16B 50/00, G16H 70/60, H04L 51/216

(54) **GRAPH REPRESENTATIONS**

(30) Priority: 14.08.2023 IN 202311054601
(71) Applicant: Fujitsu Limited, Kawasaki-shi, Kanagawa 211-8588 (JP)
(72) Inventor: MONDAL, Arnab Kumar, 560037 Bangalore (IN); NANDY, Jay, 560037 Bangalore (IN)
(74) Representative: Haseltine Lake Kempner LLP

(57) **Abstract**

A computer-implemented method comprising performing a training process, the training process comprising: using a student graph neural network, GNN, to extract a pair of first representations from a pair of training graphs, respectively; computing a disagreement between the first representations; using at least one teacher GNN to extract a pair of second representations from the pair of training graphs, respectively; computing a perceptual distance between the second representations; comparing the disagreement between the first representations with a target disagreement which is based at least in part on the perceptual distance between the second representations; and adjusting at least one weight of the student GNN based on the comparison.

## Description

The present invention relates to extracting representations from graphs, and in particular to a computer-implemented method, a computer program, and an information programming apparatus.

Annotating graphical inputs (i.e. graphs) is often expensive and time-consuming and is sometimes not realistically possible on a large scale. Instead of annotating/labelling graphs, extracting informative representations from unlabeled graphs may be useful. For example, extracting informative representations from unlabeled graphs may be an inexpensive and time-saving solution to the problem of needing labeled data to train systems for tasks involving graphs.

Neural networks are commonly employed to learn representations using self-supervised learning techniques. For example, graph neural networks may be used to learn representations from graphs. Such representations may be used for downstream tasks in various real-world domains, for example any of the following non-exhaustive list: (1) in the medical domain (a) for drug-discovery, (b) to detect disease outbreaks; (2) in chemistry, for predicting chemical properties of unknown compounds; (3) in social-networks for (a) controlling user privacy, (b) detecting spam emails; and (4) in a navigation system, to find efficient routes based on current traffic patterns.

In light of the above, it is desired to effectively extract representations from graphs.

According to an embodiment of a first aspect there is disclosed herein a computer-implemented method comprising performing a training process, the training process comprising: using a student/first graph neural network, GNN, to extract a pair of first representations from a pair of training graphs, respectively; computing/measuring/determining a disagreement/difference between the first representations (of the pair of first representations); using at least one (trained) teacher/second GNN to extract a pair of second representations from the pair of training graphs, respectively; computing a perceptual distance between the second representations (of the pair of second representations); comparing the disagreement/difference between the first representations with a target disagreement/difference which is based at least in part on the perceptual distance between the second representations; and (training the student GNN by) adjusting at least one weight of the student/first GNN based on the comparison (of the disagreement/difference with the target disagreement/difference) (to bring the disagreement/difference to or towards the target disagreement/difference).

The training process may comprise generating one training graph of the pair of training graphs by performing at least one augmentation on the other training graph of the pair of training graphs.

The training process may comprise generating one of the pair of training graphs by performing a first at least one augmentation on an original graph and generating the other of the pair of training graphs by performing a second at least one augmentation on the original graph (wherein the first and second at least one augmentations are different).

The augmentation may comprise at least one of: removing at least one node; adding at least one node; removing at least one edge; and adding at least one edge.

Using the student GNN to extract the pair of first representations from the pair of training graphs, respectively, may comprise using two instances of the student GNN with shared parameters.

Using the teacher GNN to extract the pair of second representations from the pair of training graphs, respectively, may comprise using two instances of the teacher GNN with shared parameters.

Computing the disagreement between the first representations may comprise computing a Euclidean distance (an L2 distance) between the first representations.

The training process may comprise: using each of a plurality of (said) teacher GNNs to extract a pair of second representations from the pair of training graphs, respectively; for each pair of second representations, computing a perceptual distance between the second representations of the pair; and computing an average of the plurality of perceptual distances, and the target disagreement may be based at least in part on the average of the plurality of perceptual distances.

Using a said teacher GNN to extract a said pair of second representations from the pair of training graphs, respectively, may comprise using two instances of the said teacher GNN with shared parameters.

The training process may comprise, before using the at least one teacher GNN to extract the pair of second representations, training the at least one teacher GNN.

Training the at least one teacher GNN may comprise using a contrastive learning-based training method and/or a discrepancy-based self-supervised learning-based training method and/or a predictive or prediction-based training method.

Computing the perceptual distance between the second representations (of the/each pair) may comprise computing a Euclidean distance (an L2 distance) between the second representations.

Computing the perceptual distance between the second representations and/or the disagreement between the first representations (of the/each pair) may comprise computing a Euclidean distance (an L2 distance) between (normalized) hidden representation vectors of at least one layer in each of the first/second representations.

Computing the L2 distance may comprise, for each of the first/second representations, performing a pooling operation on at least one layer of the first/second representation concerned to generate a concatenated representation of the first/second representation or of the at least one layer.

Computing the perceptual distance between the second representations and/or the disagreement between the first representations (of the/each pair) may comprise, for each of the first/second representations, performing a pooling operation on each/at least one layer of the first/second representation concerned to generate a concatenated representation of the first/second representation.

Computing the perceptual distance between the second representations and/or the disagreement between the first representations (of the/each pair) may comprise, for each of the first/second representations, performing a pooling operation on each/at least one layer of the first/second representation concerned to generate a concatenated representation of the first/second representation and computing the L2 distance between the generated concatenated representations.

The pair may be a first pair comprising a reference training graph and an augmented version of the reference training graph, the training process may further comprise performing the first and second representations extraction and the disagreement and perceptual distance computation steps based on a second pair of training graphs, the second pair of training graphs may comprise the reference graph and another reference graph (which is not an augmented version of the reference graph), comparing the disagreement with the target disagreement may comprise comparing a first (absolute) difference between the disagreements corresponding to the first and second pairs with a second (absolute) difference between the perceptual distances corresponding to the first and second pairs, and adjusting the at least one weight may comprise adjusting the at least one weight based on the comparison between the first and second differences.

The adjustment may be to bring the first difference at least above the second difference.

If the second difference is below a user-defined threshold, the adjustment may be to bring the first difference at least above the user-defined threshold.

The target disagreement may be based at least in part on (a difference between the training graphs in accordance with or a task-based target disagreement between the first representations in accordance with a use of the student GNN in) a (graph representation-extraction) task.

The target disagreement may include a task-based term based on a task-based target disagreement between the first representations in accordance with (use of the student GNN in) a (graph representation-extraction) task.

The training process may comprise comparing the disagreement with a further target disagreement based on a graph representation-extraction task, and adjusting at least one weight of the student GNN may comprise adjusting the at least one weight based on a comparison of the disagreement with the further target disagreement.

The training process may comprise comparing the disagreement with a further target disagreement based on a task-based target disagreement between the first representations in accordance with a graph representation-extraction task, and adjusting at least one weight of the student GNN may comprise adjusting the at least one weight based on comparison of the disagreement with the further target disagreement.

The training process may comprise comparing the disagreement with a further target disagreement based on a difference between the training graphs in accordance with a graph representation-extraction task, and adjusting at least one weight of the student GNN may comprise adjusting the at least one weight based on comparison of the disagreement with the further target disagreement.

Comparing the disagreement between the first representations with a target disagreement may comprise computing an error/loss between the disagreement and the target disagreement.

The training process may comprise comparing at least one of the first representations with a target representation which is based on a (graph representation-extraction) task, and adjusting at least one weight of the student GNN based on the comparison.

The computer-implemented method may comprise performing, after the training process, a (graph representation-extraction) task-based training process to train the student GNN for a (graph representation-extraction) task.

The (graph representation-extraction) task may comprise any of: extracting graph representations from graphs which represent molecules (to predict the molecules' properties); extracting graph representations from graphs representing interactions between users and products (to predict (at least one link/relation indicating) a level of interest of a user in a product); extracting graph representations from graphs representing locations of cases of a disease (to predict/detect disease outbreaks); extracting graph representations from graphs representing (road) traffic patterns (to find a route); and extracting graph representations from graphs representing emails and messages (to classify/detect at least one spam email or message).

The task may comprise any of: predicting a link in a graph representing users and products and interactions therebetween to predict whether a user will be interested in a product; classifying a graph representing a molecule (to indicate whether it will be useful for further experimental (wet lab) testing); classifying a node in a graph in which nodes represent emails and/or messages and edges represent the similarity between the emails and/or messages, the classification comprising a classification as as a spam email or not a spam email; classifying a node in a graph in which nodes represent geographical areas and links represent a measure of connectivity between the geographical areas concerned, the classification comprising a classification as a geographical area having a disease outbreak or a geographical area not having a disease outbreak.

The target disagreement may be based in part on a difference (an edit distance) between the pair of training graphs concerned

The computer-implemented method may comprise performing, after the training process, a (graph representation-extraction) task-based training process to train the student GNN for a (graph representation-extraction) task comprising any of: extracting graph representations from graphs which represent molecules (to predict the molecules' properties); extracting graph representations from graphs representing interactions between users and products (to predict (at least one link/relation indicating) a level of interest of a user in a product); extracting graph representations from graphs representing locations of cases of a disease (to predict/detect disease outbreaks); extracting graph representations from graphs representing (road) traffic patterns (to find a route); and extracting graph representations from graphs representing emails and messages (to classify/detect at least one spam email or message).

The training process may comprise: using the student GNN to extract a group of first representations from a group of training graphs (including the pair of training graphs), respectively; computing a disagreement between the first representations of each pair of first representations among the group of first representations (including the pair of first representations); using the at least one teacher GNN to extract a group of second representations from the group of training graphs (including the pair of training graphs), respectively; computing a perceptual distance between the second representations of each pair of second representations among the group of second representations (including the pair of second representations); for each pair of training graphs: comparing the disagreement between the first representations of the corresponding pair of first representations with a target disagreement which is based at least in part on the perceptual distance between the second representations of the corresponding pair of second representations; and adjusting at least one weight of the student GNN based on the comparison.

The computer-implemented method may comprise iterating/repeating the training process a plurality of times using different respective pairs/groups of training graphs.

The computer-implemented method may comprise iterating/repeating the training process until (an iteration in which) the error converges or is below an error threshold.

The computer-implemented method may comprise iterating/repeating the training process until (a predefined number of successive iterations in which) the error is below an error threshold.

The computer-implemented method may comprise iterating/repeating the training process a predefined number of times.

The computer-implemented method may comprise using the (trained) student graph (after training) to perform a (graph representation-extraction) task comprising any of: extracting graph representations from graphs which represent molecules (to predict the molecules' properties); extracting graph representations from graphs representing interactions between users and products (to predict (at least one link/relation indicating) a level of interest of a user in a product); extracting graph representations from graphs representing locations of cases of a disease (to predict/detect disease outbreaks); extracting graph representations from graphs representing (road) traffic patterns (to find a route); and extracting graph representations from graphs representing emails and messages (to classify/detect at least one spam email or message).

According to an embodiment of a second aspect there is disclosed herein a computer program which, when run on a computer, causes the computer to carry out a method comprising performing a training process, the training process comprising: using a student/first graph neural network, GNN, to extract a pair of first representations from a pair of training graphs, respectively; computing/measuring/determining a disagreement/difference between the first representations (of the pair of first representations); using at least one (trained) teacher/second GNN to extract a pair of second representations from the pair of training graphs, respectively; computing a perceptual distance between the second representations (of the pair of second representations); comparing the disagreement/difference between the first representations with a target disagreement/difference which is based at least in part on the perceptual distance between the second representations; and (training the student GNN by) adjusting at least one weight of the student/first GNN based on the comparison (of the disagreement/difference with the target disagreement/difference) (to bring the disagreement/difference to or towards the target disagreement/difference).

According to an embodiment of a third aspect there is disclosed herein an information processing apparatus comprising a memory and a processor connected to the memory, wherein the processor is configured to perform a training process, the training process comprising: using a student/first graph neural network, GNN, to extract a pair of first representations from a pair of training graphs, respectively; computing/measuring/determining a disagreement/difference between the first representations (of the pair of first representations); using at least one (trained) teacher/second GNN to extract a pair of second representations from the pair of training graphs, respectively; computing a perceptual distance between the second representations (of the pair of second representations); comparing the disagreement/difference between the first representations with a target disagreement/difference which is based at least in part on the perceptual distance between the second representations; and (training the student GNN by) adjusting at least one weight of the student/first GNN based on the comparison (of the disagreement/difference with the target disagreement/difference) (to bring the disagreement/difference to or towards the target disagreement/difference).

Features relating to any aspect/embodiment may be applied to any other aspect/embodiment.

Reference will now be made, by way of example, to the accompanying drawings, in which:
Figure 1 is a diagram illustrating a comparative method;
Figure 2 is a diagram illustrating a comparative method;
Figure 3 is a diagram illustrating a comparative method;
Figure 4 is a diagram illustrating a training process;
Figure 5 is a diagram illustrating a training process;
Figure 6 is a diagram illustrating a training process;
Figure 7 is a diagram illustrating a training process;
Figure 8 is a diagram illustrating a training process;
Figure 9 is a diagram illustrating a training process;
Figure 10 is a diagram useful for understanding the invention;
Figure 11 is a table of results;
Figure 12 is a diagram illustrating an example implementation;
Figure 13 is a diagram illustrating an example implementation;
Figure 14 is a diagram illustrating an example implementation; and
Figure 15 is a diagram illustrating an apparatus.

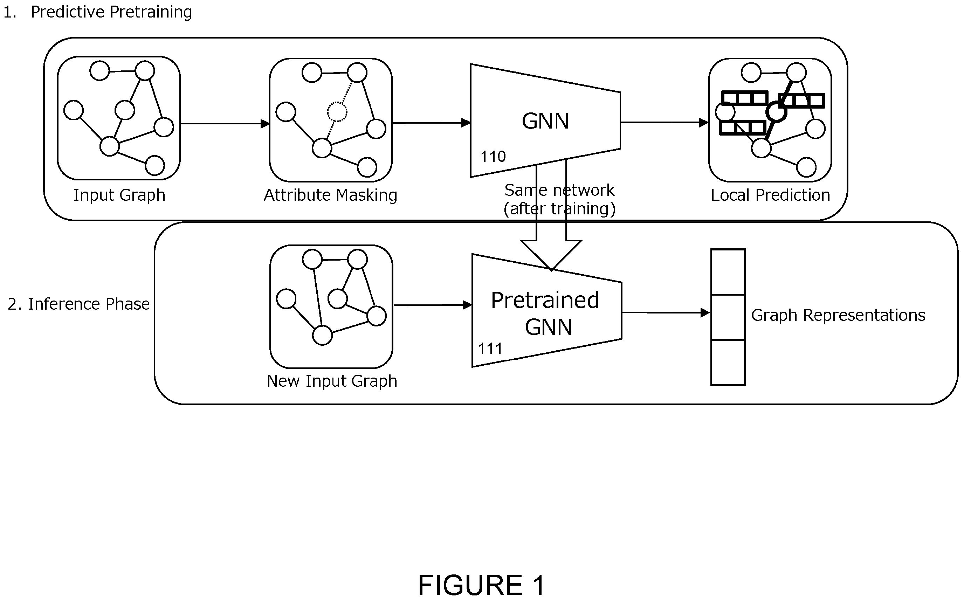

Figure 1 illustrates a first comparative method. The first comparative method is a predictive or prediction-based method for training a graph neural network (GNN) to extract representations from graphs. The first comparative method is illustrated in "1. predictive pretraining". Attribute masking is carried out on an input graph. In other words, at least one node and/or at least one edge is "masked" or removed from the input graph. Then, a GNN is used to predict the masked attribute(s). The GNN is updated based on the difference between the predicted attribute(s) and the actual masked attribute(s). In this way, the GNN is trained. Illustrated at "2. Inference phase" is the subsequent use of the GNN after training to extract graph representations from an input graph.

A problem with the first comparative method is that GNNs trained in this way do not tend to successfully predict the global structure of graphs.

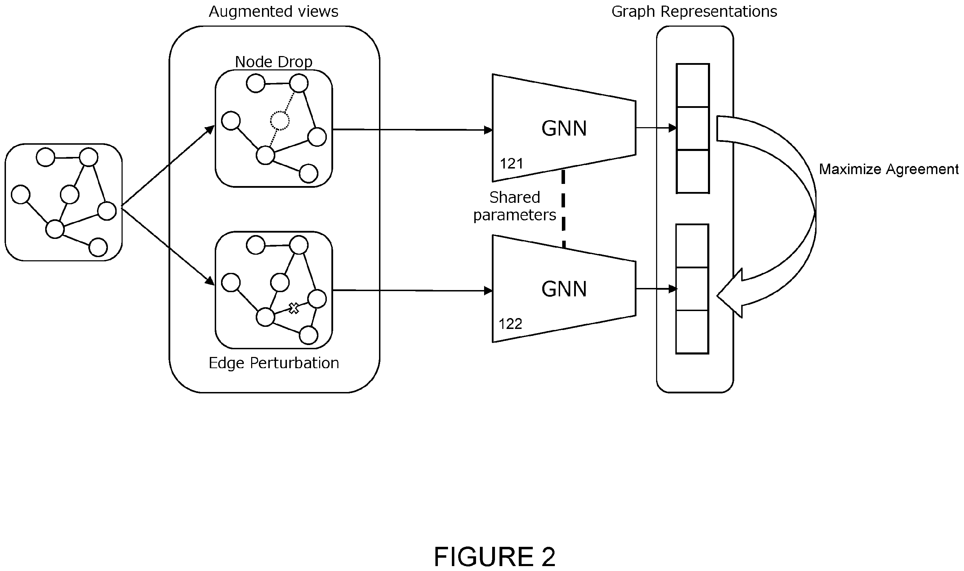

Figure 2 illustrates a second comparative method. The second comparative method is a contrastive learning-based method for training a graph neural network (GNN) to extract representations from graphs. In the second comparative method, an input graph is augmented (e.g. by removal of a node or removal/translation of an edge). A GNN is used to extract representations from two augmentations of the input graph (as illustrated, two instances of the GNN with shared parameters may be used). The GNN is then adjusted to maximize agreement between the two extracted representations.

A problem with the second comparative method is that some perturbations/augmentations of a graph (such as editing nodes/edges) can significantly change graph properties. For example, it may be the case that two augmentations of an input graph may have very different properties (e.g. in the applicable domain) even though they are considered in the second comparative method to be similar.

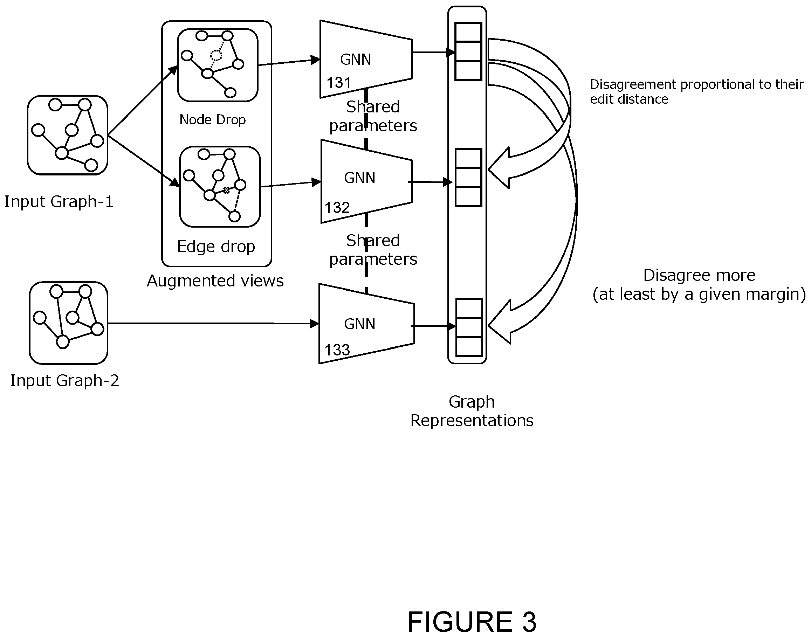

Figure 3 illustrates a third comparative method. The third comparative method is a discrepancy-based self-supervised learning (D-SLA) method for training a graph neural network (GNN) to extract representations from graphs. In the third comparative method, a first input graph is augmented to produce two augmentations (for example in the same way as the input graph is augmented in the second comparative method). A GNN is used to extract representations from the two augmentations of the first input graph. The GNN is adjusted to bring the disagreement between the representations to or towards a loss amount proportional to an edit distance between the augmentations. An edit distance may comprise/be based on, for example, the minimum number of augmentations required to obtain one augmented version of the input graph starting from the other augmentation of the input graph.

The third comparative method further comprises using the GNN to extract a representation from a second input graph (which is not simply an augmentation of the first input graph). The adjustment of the GNN is then also aimed at bringing the disagreement between the representation of the second input graph and the representation of an augmentation of the first input graph to or towards a value greater than the disagreement between the representations of the augmentations of the first input graph.

In other words, the GNN is trained to make the first disagreement between the representations of the augmentations of the first input graph proportional to the edit distance between the augmentations, and to make the representation of the second input graph and a representation of an augmentation of the first input graph disagree "more" than the representations of the augmentations of the first input graph (at least by a given margin).

A problem with the third comparative method is that the edit distance between two augmentations of an input graph (or between a graph and its augmentation/perturbed variation) cannot capture the accurate semantic difference between those graphs. Another problem is that computing an edit distance between any two arbitrary graphs is NP-hard.

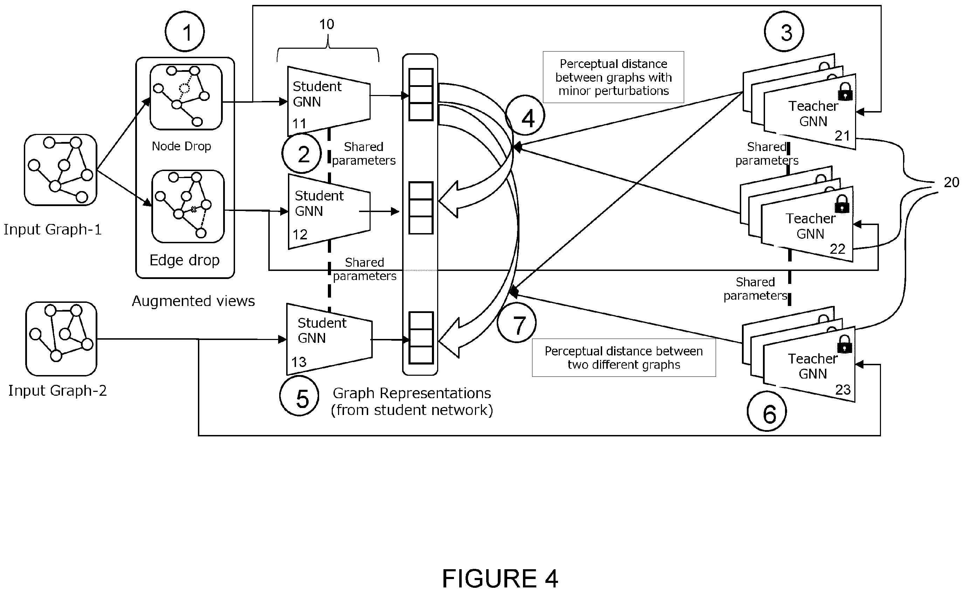

Figure 4 illustrates an example implementation of a training process. The training process in Figure 4 is for training a GNN to extract representations from graphs.

At circle 1, an input graph (input graph 1) is augmented, e.g. by any of: removing at least one node; adding at least one node; removing at least one edge; and adding at least one edge. In this way, two augmentations of input graph 1 are generated, which may be referred to as first and second input graphs. The augmentations may be referred to as augmented or perturbed versions/variations. Instead of performing two separate augmentations, one augmentation may be performed and the original input graph 1 may be used for the further procedure in place of the second augmented version of input graph 1. That is, instead of using two augmented versions of input graph 1 as the first and second input graphs, an augmented version and the original input graph 1 may be used as the first and second input graphs.

At circle 2, a GNN 10 used to extract representations from the first and second input graphs, respectively. The GNN 10 may be referred to as a first GNN or a student GNN. As illustrated, two instances 11 and 12 of the student GNN 10 are used for the first and second input graphs, respectively. The student GNNs 11 and 12 have shared parameters - in other words, the student GNNs 11 and 12 have the same parameters as each other - and may therefore be considered the same GNN. The student GNNs 11 and 12 may be referred to collectively as a student GNN 10.

At circle 3, a different GNN 20 is used to extract representations from the first and second input graphs, respectively. The GNN 20 may be referred to a second GNN or a teacher GNN. As illustrated, two instances 21 and 22 of the teacher GNN 20 are used for the first and second input graphs, respectively. The teacher GNNs 21 and 22 have shared parameters - in other words, the teacher GNNs 21 and 22 have the same parameters as each other - and may therefore be considered the same GNN. The teacher GNNs 21 and 22 may be referred to collectively as a teacher GNN 20. The teacher GNN 20 is a trained GNN, i.e. the teacher GNN 20 has already undergone a training process.

At circle 4, a perceptual distance between the representations of the first and second input graphs extracted by the teacher GNN 20 is computed. The perceptual distance may be or comprise an L2 distance and is described in more detail later. The disagreement between the representations of the first and second input graphs extracted by the student GNN 10 is computed which may be or comprise an L2 distance. This disagreement is compared with a target disagreement which is based at least in part on the perceptual distance. The student GNN 10 is adjusted to bring the disagreement to or towards the target disagreement (in other words, to minimize an error/loss calculated between the disagreement and the target disagreement). The first and second GNNs 10 and 20 may be referred to with the labels "student" and "teacher" because the first GNN 10 may be considered to be "learning from" the second GNN 20 due to the comparison of the disagreement with the perceptual distance. The labels "student" and "teacher" may be replaced with "first" and "second".

At circle 5 the student GNN 10 is used to extract a representation from a different input graph, input graph 2.

At circle 6 the teacher GNN 20 is used to extract a representation from input graph 2.

At circle 7 a perceptual distance between the representation of the first input graph extracted by the teacher GNN 20 and the representation of input graph 2 extracted by the teacher GNN is computed. The perceptual distance may be or comprise an L2 distance. A disagreement between the representations of the first input graph and input graph 2 extracted by the student GNN 10 is computed which may be or comprise an L2 distance. This disagreement is compared with another target disagreement which is based at least in part on the perceptual distance between the representations of the first input graph and input graph 2 extracted by the teacher GNN 20. The student GNN 10 is adjusted to bring the disagreement to or towards this target disagreement (in other words, to minimize an error/loss calculated between the disagreement and the target disagreement).

As illustrated in Figure 4, the processing of circles 5-7 may be carried out together with (i.e. in the same training process as) the processing in circles 1-4. In this case, for example, a further instance 13 of the student GNN 10 and a further instance 23 of the teacher GNN 20 may be used, as shown. Parameters are shared (i.e. are the same) between the instances 11-13 of the student GNN and the instances may be referred to collectively as a student GNN 10. Parameters are shared (i.e. are the same) between the instances 21-23 of the teacher GNN and the instances may be referred to collectively as a teacher GNN 20.

A training process may comprise the processing of circles 1-4 and not the processing of circles 5-7. Or a training process may comprise the processing of circles 5-7 and not the processing of circles 1-4. It will be appreciated that the processing of circles 2-4 is the same as that of circles 5-7 except that the input graphs used are different. In this way, it may be said that the training process comprises the processing of circles 2-4 based on two input images - these input images may be two different and separate input images, or they may be such that one input image is an augmentation or augmented version of the other, or they may be such that both input images are different augmentations or augmented versions of another input image.

A method in an implementation example comprises iterating the training process to train the student GNN 10 (to bring the disagreement to or towards the target disagreement). Different iterations comprise using different pairs of input images - a method may comprise any of an iteration in which the processing of circles 1-4 is used, an iteration in which the processing of circles 5-7 is used, and an iteration in which the processing of circles 1-7 is used.

In Figure 4, further teacher GNNs are shown behind the instances of the teacher GNN 20. Instead of just the teacher GNN 20 extracting representations from the two input graphs concerned, a plurality of different teacher GNNs may be used. That is, a plurality of teacher GNNs may be used to extract a plurality of pairs of representations from the said pair of input images. Then, a plurality of perceptual distances may be computed corresponding to each teacher GNN. The target disagreement in such a training process may be based at least in part on an average (i.e. mean) of those perceptual distances.

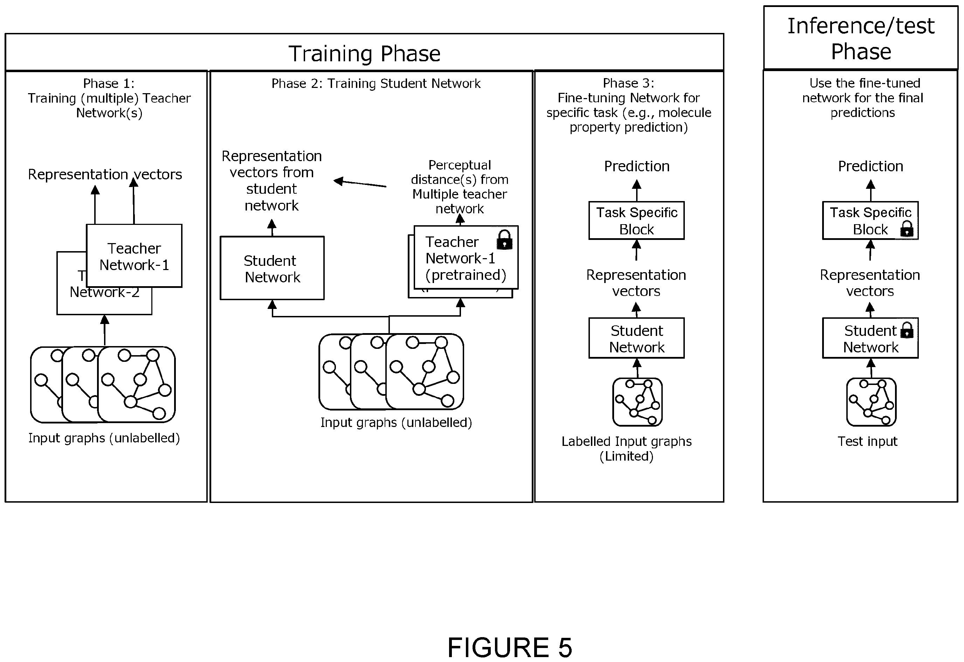

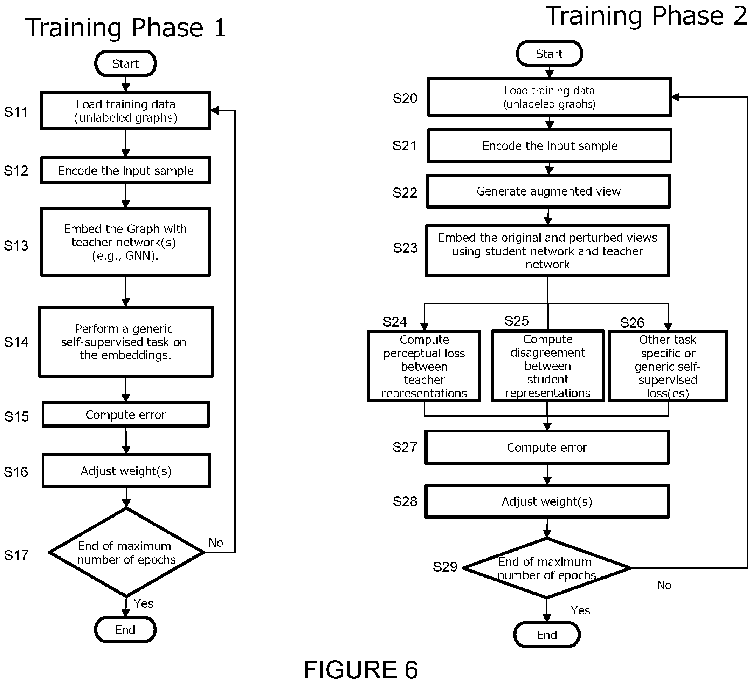

Figure 5 illustrates a training phase and an inference or test phase according to an implementation example. The training phase comprises phases 1, 2, and 3. The training phases and the inference or test phase are described in further detail in Figures 6-8. The training phase and test phase may be considered a training process. The training phase or just phase 2 or just phases 2 and 3 or just phases 1 and 2 may be considered a training process.

Training phase 1 comprises training at least one teacher GNN. This training may be carried out using comparative method 3, or any other comparative method, or any other training method. A plurality of teacher GNNs may be trained in training phase 1.

Training phase 2 comprises training a student GNN. For example, the training process described with respect to Figure 4 may be iterated using the teacher GNN(s) trained in training phase 1.

Training phase 3 comprises further training for a specific task - i.e. a task for which the student GNN is intended to be used. This task may be referred to as a downstream task. This training phase may be considered to comprise fine-tuning the student GNN trained in training phase 2.

The inference or test phase comprises using a test input graph and using the trained student GNN to extract a representation for testing/verification.

Figure 6 illustrates methods according to training phases 1 and 2, respectively.

The method according to training phase 1 comprises steps S11-S17. Step S 11 comprises loading training data in the form of input graphs or "training graphs". The training graphs are unlabeled. One training graph or two, or more than two, may be loaded. In a running example, multiple training graphs are loaded in the form of a batch of training graphs.

Step S12 comprises encoding the input sample, i.e. encoding at least one training graph. In the running example, the batch of training graphs are encoded.

Step S13 comprises embedding a training graph with at least one of the at least one teacher GNN to be trained. In other words, step S13 comprises extracting a representation from the training graph with the at least one teacher GNN.

Step S14 comprises performing a self-supervised task on the embedding(s)/representation(s). That is, Step S14 comprises performing processing corresponding to a training method, for example any of the first to third comparative methods. For example, step S14 may comprise using the at least one teacher GNN to extract a representation from another training graph and computing the disagreement between the two representations.

Step S15 comprises computing an error. The error is computed in accordance with the training method (e.g. any of the first to third comparative methods) used. For example, step S15 may comprise determining an edit distance between two training graphs from which the teacher GNN extracted representations, and computing an error based on the disagreement between the representations and the edit distance between the graphs.

Step S15 may comprise a comparison rather than a direct error computation. For example, step S15 may comprise comparing the edit distance to the disagreement.

Step S16 comprises adjusting at least one weight of the teacher GNN, i.e. to reduce the computed error. For example, a weight may be adjusted to bring the disagreement to or towards proportionality with the edit distance.

Step S17 comprises determining whether the maximum number of epochs has been reached. If yes then the method ends. If no then the method proceeds to step S11 and another at least one training graph is loaded. The method is iterated in this way. Alternatively, an error convergence may be determined to decide whether to perform a further iteration or end the method. For example, it may be determined whether the error has been below a threshold for a predetermined number of iterations. This may be determined in addition to the maximum epoch determination - i.e. the method may end if either determination is true ("yes"), that is, if either the maximum number of epochs has been reached or if the error is determined to be converging/have converged.

As mentioned above, any training technique may be used to train the teacher GNN, for example any of the first to third comparative methods. The method of training phase 1 may be considered to not comprise step S11 and/or step S12 and one or both of these steps may be carried out in a pre-method process.

Training phase 2 comprises steps S20-S29. Step S20 comprises loading training data in the form of input graphs or "training graphs". The training graphs are unlabeled. One training graph or two, or more than two, may be loaded. In a running example, multiple training graphs are loaded in the form of a batch of training graphs.

Step S21 comprises encoding the input sample, i.e. encoding the training graph loaded (or one of the training graphs loaded). In the running example, the batch of training graphs are encoded.

Step S22 comprises generating an augmented view of the training graph. The augmentation may comprise at least one of: removing at least one node; adding at least one node; removing at least one edge; and adding at least one edge. The augmented view may be referred to as an augmented version or perturbed view/variation.

Step S23 comprises embedding the training graph and the perturbed view with the student GNN to be trained to generate/extract two representations which may be referred to herein as a pair of first representations. Step S23 further comprises embedding the training graph and the perturbed view with the trained teacher GNN (i.e. trained in training phase 1) to generate/extract two representations which may be referred to herein as a pair of second representations.

Step S24 comprises computing the perceptual loss or distance between the second representations (or "teacher representations"). As described later, the perceptual distance may be or comprise an L2 distance.

Step S25 comprises computing the disagreement between the first representations (or "student representations"). As described later, the disagreement may be or comprise an L2 distance. This disagreement may be referred to as a student perceptual distance and the perceptual distance between the second representations may be referred to as a teacher perceptual distance.

Step S26 comprises computing at least one other loss term. For example, this may comprise a task-specific loss term and/or e.g. an edit distance-based loss term, for example. Step S26 is optional.

Step S27 comprises computing an error. For example, an error is computed between the disagreement computed in step S25 and a target disagreement which is based at least in part on the perceptual distance computed in step S24 and the at least one loss term computed in step S26 if applicable.

Step S27 may comprise a comparison rather than a direct error computation. For example, step S27 may comprise comparing the disagreement to the target disagreement.

Step S28 comprises adjusting at least one weight of the student GNN to reduce the error, i.e. to bring the disagreement computed in step S25 to or towards the target disagreement. In a running example, all the weights of the student GNN are adjusted.

Step S29 comprises determining whether the maximum number of epochs has been reached. If yes then the method ends. If no then the method proceeds to step S20 and another training graph is loaded. The method is iterated in this way. Alternatively, an error convergence may be determined to decide whether to perform a further iteration or end the method. For example, it may be determined whether the error has been below a threshold for a predetermined number of iterations. This may be determined in addition to the maximum epoch determination - i.e. the method may end if either determination is true ("yes"), that is, if either the maximum number of epochs has been reached or if the error is determined to be converging/have converged.

The method of training phase 2 may be considered to not comprise step S20 and/or step S21. One or both of these steps may be considered to be carried out in a pre-method process. For example, all of the training graphs may be loaded (and encoded) before the method of training phase 2.

For a given iteration, the training graph and the perturbed version may be referred to as a pair of training graphs (or a pair of input graphs). In at least one iteration instead of a training graph and its perturbed version, two different training graphs may be used as the pair of training graphs and the method may not comprise step S22 for that at least one iteration. In a running example, an iteration (e.g. some or every iteration) may comprise using a first pair of training graphs comprising a reference graph and its perturbed variation, and a second pair of training graphs comprising the reference graph and a different graph which is not a perturbed variation. That is, the reference graph may be shared/common between the two pairs. In this case, a disagreement and a perceptual distance may be computed corresponding to each pair, and a comparison and weight adjustment may be carried out for each pair (the comparison and weight adjustment as described above). The weight adjustment may be carried out in one step for both pairs of training graphs. In this running example the reference graph and its perturbed variation may be considered positive training examples or a positive pair and the reference graph and the different graph may be considered negative training examples or a negative pair.

At the end of the method of training phase 2 a trained student GNN is provided.

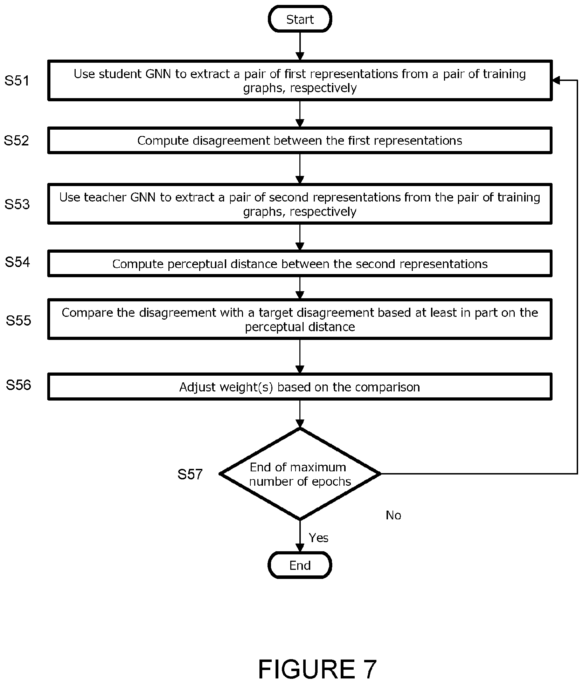

Figure 7 illustrates another implementation example of a training process. It will be appreciated that the method in Figure 7 could be made to correspond with the method in Figure 6 (training phase 2) with some adjustments.

The method in Figure 7 comprises steps S51-S57.

Step S51 comprises using a student/first GNN to extract a pair of first representations from a pair of training graphs, respectively.

Step S52 comprises computing/measuring/determining a disagreement/difference between the first representations (of the pair of first representations).

Step S53 comprises using at least one (trained) teacher/second GNN to extract a pair of second representations from the pair of training graphs, respectively.

Step S54 comprises computing a perceptual distance between the second representations (of the pair of second representations).

Step S55 comprises comparing the disagreement/difference between the first representations with a target disagreement/difference which is based at least in part on the perceptual distance between the second representations.

Step S56 comprises adjusting at least one weight of the student/first GNN based on the comparison, for example to bring the disagreement/difference to or towards the target disagreement/difference. In a running example all the weights of the student GNN are updated.

Step S57 comprises determining whether to perform another iteration using a different pair of training graphs (return to step S51) or end the method and the considerations described above with reference to steps S17 and S29 may be considered to apply to step S57.

Steps S51-S56 or steps S51-S57 may be referred to as a training process.

In the above description of Figures 6 and 7, adjusting at least one weight may be referred to as updating at least one network parameter.

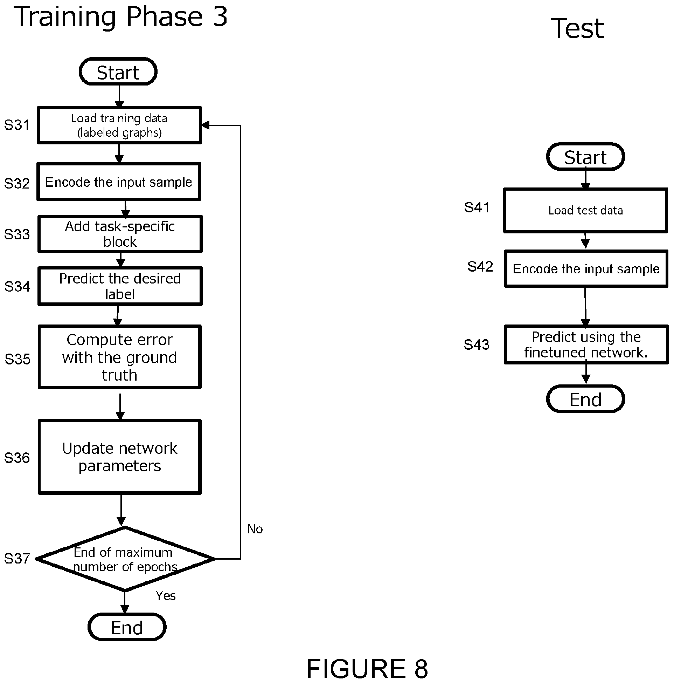

Figure 8 illustrates two methods corresponding to the training phase 3 and the test phase illustrated in Figure 5, respectively.

The method of training phase 3 comprises steps S31-S37 and is for training the student GNN for a specific task (downstream task).

Step S31 comprises loading training data, for example at least one training graph. In a running example a plurality of training graphs are loaded, for example in the form of a (training) batch.

Step S32 comprises encoding the input sample (the at least one training graph). Considerations described above with reference to steps S12 and S21 may apply to step S32.

Step S33 comprises adding a task-specific block to the trained student GNN. For example, a classification head (as the task-specific block) may be added onto the student GNN so that the student GNN can be used (and trained specifically for/finetuned for) the task. This step may be considered carried out as a preliminary step before the method of training phase 3.

Step S34 comprises predicting at least one desired label. This is an example of a task. The at least one label may be of a node and/or an edge and/or the entire graph. The at least one label may be referred to as at least one class label. Step S34 may comprise predicting a node or an edge in a graph instead or in addition. A number of example tasks are described later. Step S34 comprises using the student GNN trained in training phase 2 to make the prediction.

Step S35 comprises computing the error between the predicted label and the ground truth (the actual label). If an edge or node has been predicted, for example, step S35 may comprise computing an error between the prediction and the ground truth (actual) node or edge. Step S35 comprises, in general, comparing the prediction with the ground truth.

Step S36 comprises updating network parameters of the student GNN, i.e. adjusting at least one weight. This adjustment is to reduce the error, i.e. to bring the prediction to or towards the ground truth. In a running example all of the weights of the student GNN are updated.

Step S37 comprises determining whether to perform another iteration (returning to step S31) or end the method. Considerations described above with reference to steps S17 and S29 may apply to step S37.

The method according to the test phase comprises steps S41-S43. Step S41 comprises loading test data, i.e. a test graph. Step S42 comprises encoding the input sample (the test graph). Considerations described above with reference to loading and encoding steps may apply to steps S41 and S42. Step S43 comprises generating a prediction using the student GNN that has been trained (according to training phase 2) and/or further trained or fine-tuned according to training phase 3.

The training phases 1-3 and the test phase may be considered part of an overall method. Regarding training the student GNN for a specific (downstream) task, it will be appreciated that such training may be carried out as part of the training phase 2 and/or as the separate training phase 3. The training for a specific task is not essential. That is, method aspects disclosed herein may be or comprise a method as described with respect to training phase 2 (Figure 6 or 7) (and the target disagreement in the method of training phase 2 may not depend on a task-specific loss/disagreement).

The method of training phase 1 is not essential and method aspects disclosed herein may be or comprise a method as described with respect to training phase 2 (Figure 6 or 7) and may utilize teacher GNN(s) already trained.

The test phase is also not essential. That is, method aspects disclosed herein may be or comprise a method as described with respect to training phase 2 (Figure 6 or 7).

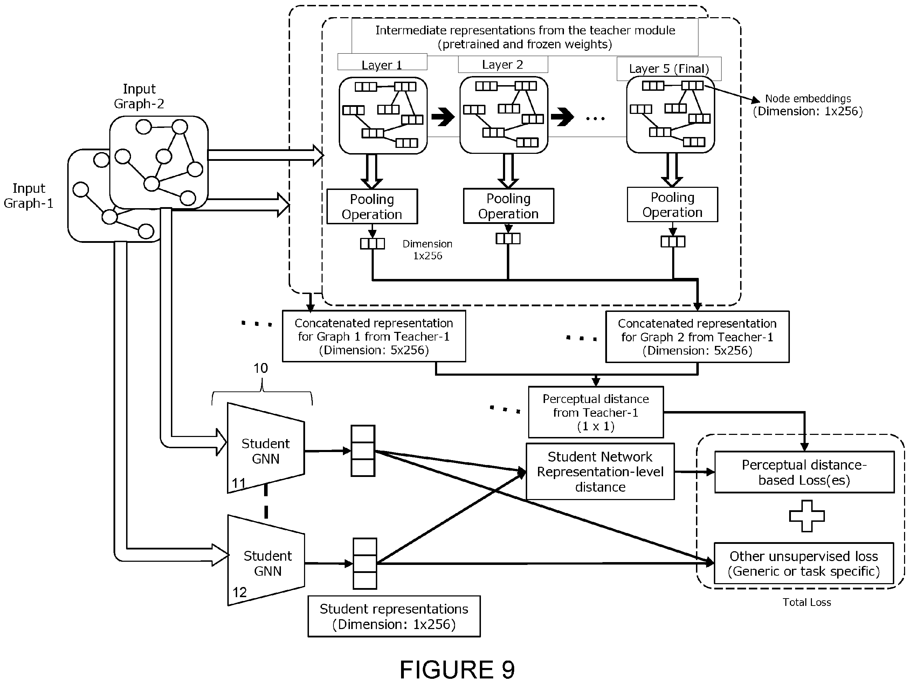

Figure 9 is a schematic diagram illustrating a training process according to an implementation example. The training process may correspond to the training process described with respect to Figure 4 and/or Figure 6 (training phase 2) and/or Figure 7. In Figure 9 some example dimensions are illustrated. These dimensions are for aiding understanding and are not exhaustive or limiting.

As illustrated, a student GNN 10 (to be trained) is used to extract a pair of first representations from a pair of training graphs (input graph 1 and input graph 2). A teacher GNN is used to extract a pair of second representations from the pair of training graphs.

In this implementation example, the L2 distance between the second representations is computed. Considering the second representation corresponding to input graph 1, which may be referred to herein as representation 1, a pooling operation is performed on each intermediate layer of representation 1. For example, max pooling may be used (similarly, mean pooling or hierarchical pooling may be used). In this example there are 5 layers, but there may be any number of layers. The number of layers is not necessarily the same as the number of layers of the corresponding teacher GNN.

As shown in Figure 9 (top right-hand-side), each node embedding in each layer has, in this example, a dimension of 1 x 256. After the pooling operations, a vector of dimension 1 x 256 is generated for each intermediate layer of representation 1. Based on the vectors generated by the pooling operations on the intermediate layers of representation 1, a concatenation is performed to generate a vector of dimension 5 x 256 (because in this example there are 5 layers).

As illustrated in Figure 9, a corresponding process is performed based on the second representation of input graph 2 (which may be referred to as representation 2) and a concatenated representation with dimension 5 x 256 is generated corresponding to representation 2.

The perceptual distance between these concatenated representations is computed as the perceptual distance between the second representations. To compute the perceptual distance between the concatenated representations the L2 distance may be computed. Similarly L1 distance or inverse of dot-products of two vectors may be computed. The L2 distance may be referred to as the Squared Euclidean or L2-Squared distance, and comprises calculating the distance between two vectors by taking the sum of the squared vector values. This may be considered (and indeed referred to, as above) as computing the L2 distance between the second representations.

The disagreement between the first representations is computed (referred to in Figure 9 as "student network representation-level distance"). Although not shown in Figure 9 the disagreement may be or comprise an L2 distance and may be computed in line with the description above of the perceptual distance computation. The disagreement is compared with a loss based at least in part on the perceptual distance between the second representations. As illustrated in Figure 9, the first representations are also compared with at least one other loss term (referred to in Figure 9 as "other unsupervised loss (generic or task specific)"). This comparison may utilize the disagreement between the first representations or the comparison may be a direct comparison of one or both of the first representations with a target representation corresponding to e.g. a task that the student GNN is to be trained for.

Rather than performing the comparison with the two at least one loss terms separately, the disagreement between the first representations may be compared with a loss which includes a term based on the perceptual distance between the second representations and/or at least one term based on a task-specific loss and/or at least one term based on a generic loss.

As indicated by the dots in Figure 9, a plurality of teacher GNNs may be used to generate a plurality of pairs of second representations, respectively. The process described above for computing the perceptual distance may be carried out for each pair of second representations. The overall perceptual distance upon which the loss term is (at least in part) based may comprise an average (i.e. mean) of the perceptual distances corresponding to each pair of second representations.

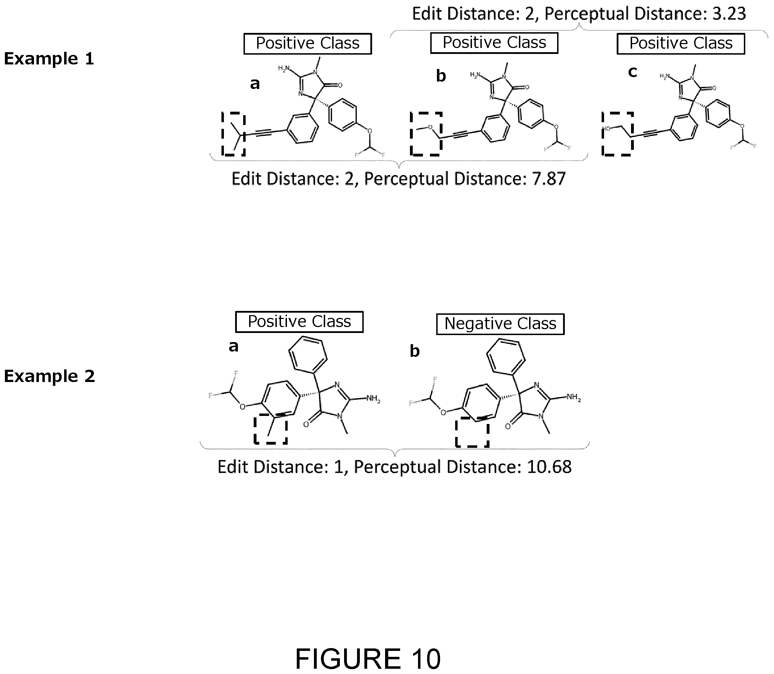

Figure 10 is a schematic diagram illustrating two examples showing the difference and relative usefulness of the edit distance and the perceptual distance. In these examples, the perceptual distance was computed using a teacher network trained using the D-SLA method.

In example 1 three molecules, a-c, are shown. The difference between the three molecules is highlighted in each case with a dashed line box. The edit distance between molecules a and b is 2, whereas the perceptual distance is 7.87. The edit distance between molecules b and c is 2 whereas the perceptual distance is 3.23. All three molecules a-c belong to the same class (positive class). Example 1 serves to illustrate that the perceptual distance between molecules of a first pair can differ significantly from the perceptual distance between molecules of a second pair even though the edit distance between the molecules of the first pair is the same as the edit distance between the molecules of the second pair. In this way, it will be appreciated that the edit distance may not effectively capture the semantic difference between graphs.

In example 2, two molecules, a and b, are shown. The difference between the molecules is highlighted with dashed lines boxes. Molecules a and b belong to different classes and the perceptual distance between them is 10.68, whereas the edit distance between them is 1. Example 2 serves to illustrate that the edit distance may be small even when the molecules have a large perceptual distance and belong to different classes.

Furthermore, considering example 1 together with example 2, it will be appreciated that the edit distances in example 1 are larger than that in example 2 even though the molecules in example 1 belong to the same class while those in example 2 belong to different classes and the perceptual distances in example 1 are smaller than that in example 2. Together, these examples serve to illustrate that the edit distance may fail to capture semantic differences between graphs.

The loss/error in the training processes described above for training the student GNN (including training phase 2) may comprise a term based on an edit distance between the pair of training graphs. In this way the training of the student GNN is based on both the structural and semantic differences between the training graphs of the pair of training graphs. The perceptual distance in any such training method may be considered to act as a regularizer, e.g. for providing a better notion of semantic differences between training graphs. For example, the loss term based on the perceptual distance may comprise a regularization term which imposes a penalty (increase loss) based on the size of the perceptual distance. In this way, it may be considered that when the teacher GNN is "incorrect" (i.e. when the perceptual distance is very different from the edit distance), the student GNN may "choose" to learn from the raw data instead, which may result in better graph representation-extraction.

In a running example in which the training graphs for an iteration comprise the first pair of training graphs comprising the reference graph and its perturbed variation, and the second pair of training graphs comprising the reference graph and the different graph which is not a perturbed variation, the loss may be computed as follows.

The perceptual distance between the second representations of the graphs of the first pair is referred to as a positive perceptual distance and the perceptual distance between the second representations of the graphs of the second pair is referred to as a negative perceptual distance. Similarly, the disagreement between the first representations of the graphs of the first pair is referred to as a positive disagreement and the disagreement between the first representations of the graphs of the second pair is referred to as a negative disagreement.

The difference between the positive and negative disagreements is compared with the difference between the positive and negative perceptual distances. There is a user-defined threshold for the difference between the positive and negative disagreements so that if the difference between the perceptual distances is below this threshold, the student network uses the raw data and may be considered to learn from the raw data instead of the second (teacher) representations. The lower limit defined by the perceptual distance may be considered to give rise to a dynamic error/loss margin. In addition, the difference between the positive and negative perceptual distances may act as a lower limit so that the weight adjustment step is to bring the difference between the positive and negative disagreements at least above this lower limit.

It will be appreciated that this implementation is somewhat in line with the contrastive or triplet loss scheme for ML training. Triplet loss is a loss function where a reference input (called an anchor) is compared to a matching input (called positive) and a non-matching input (called negative). The distance from the anchor to the positive is minimized, and the distance from the anchor to the negative input is maximized. Alternatively, a modified version of NT-Xent (Normalized Temperature-Scaled Cross-Entropy) loss may be applied as part of the comparison step by updating similarity measures for positive pairs and negative pairs using a threshold margin.

It will be appreciated that the comparison of the disagreement with the loss/error may be implemented in a number of ways as disclosed herein.

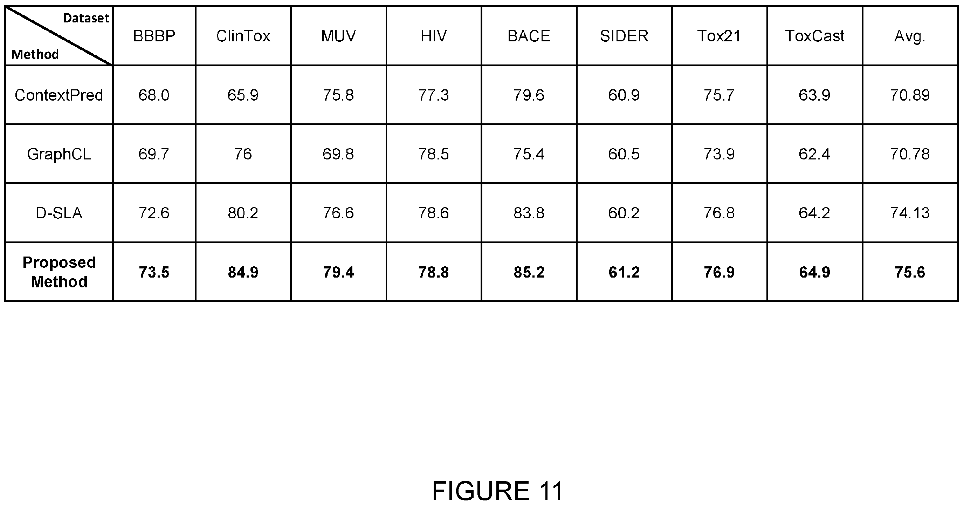

Figure 11 illustrates a table. The table comprises results (in the form of prediction accuracy) of applying three known methods and a method according to invention embodiments ("proposed method") to eight datasets. The known methods are ContextPred (Hu, Weihua, et al. "Strategies for pre-training graph neural networks." arXiv preprint arXiv:1905.12265 (2019)), GraphCL (You, Yuning, et al. "Graph contrastive learning with augmentations." Advances in neural information processing systems 33 (2020): 5812-5823), and D-SLA (Kim, Dongki, Jinheon Baek, and Sung Ju Hwang. "Graph self-supervised learning with accurate discrepancy learning." arXiv preprint arXiv:2202.02989 (2022)). The datasets are BBBP, ClinTox, MUV, HIV, BACE, SIDER, Tox21, and ToxCast (Wu, Zhenqin, et al. "MoleculeNet: a benchmark for molecular machine learning." Chemical science 9.2 (2018): 513-530).

The proposed method comprised training a teacher model using the D-SLA method using the zinc dataset (Teague Sterling and John J Irwin. Zinc 15-ligand discovery for everyone. Journal of chemical information and modeling, 55(11):2324-2337, 2015), which is a large-scale dataset containing 2 million unannotated datapoints. A student GNN was then trained according to the training process described above (i.e. as illustrated in Figure 4/6 (phase 2)/7/9) using the trained teacher model as the teacher GNN. The trained student GNN was then finetuned separately using the eight datasets BBBP, ClinTox, MUV, HIV, BACE, SIDER, Tox21, and ToxCast.

The trained and fine-tuned student GNNs were then used to carry out a classification task (a molecular property prediction task - Wu, Zhenqin, et al. "MoleculeNet: a benchmark for molecular machine learning." Chemical science 9.2 (2018): 513-530)) and the AUROC score was computed in each case.

The other methods (ContextPred, GraphCL, and D-SLA) were used to train/finetune GNNs using the zinc dataset and the eight datasets, and these trained GNNs were used to carry out the same classification task. The corresponding AUROC scores were calculated.

The AUROC scores for the known methods and for the proposed method are shown in the table in Figure 11. The average score is also shown for each method. It will be appreciated that the average score for the proposed method is higher than that of any of the known methods, and the score for each dataset is also higher than that of any of the known methods. This indicates advantages of the methods proposed herein (for example improved representation of graphs trained in accordance with the methods proposed herein) compared to known methods for training GNNs for representation extraction. That is, the graph representations extracted by a GNN trained in accordance with the methods disclosed herein may capture more information from the graph (semantic and/or structural) than other methods. The methods proposed herein may be considered advantageous for example due to the process of knowledge distillation.

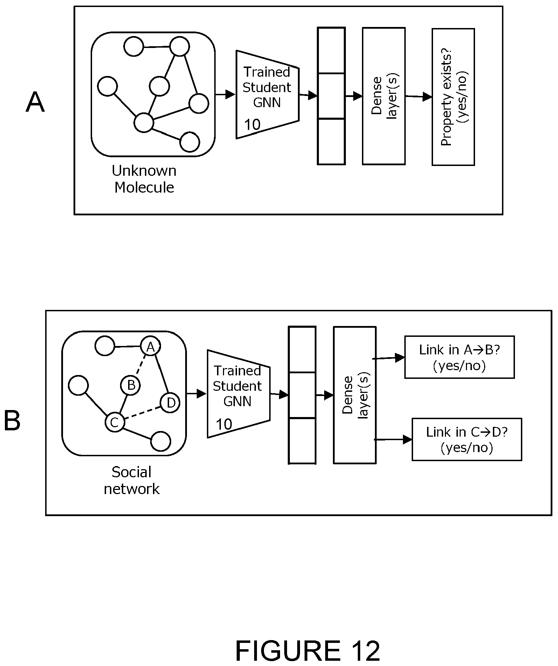

Figure 12 illustrates two example implementations A and B of the methods for training a GNN disclosed herein. In A, a student GNN 10 trained in accordance with methods disclosed herein is used for graph classification and prediction of chemical property(ies) of a molecule (e.g. an unknown molecule). That is, the student GNN 10 extracts a representation from the graph indicating the unknown molecule, and based on the representation (e.g. based on the dense layers) a prediction is made as to whether a particular property is likely to be possessed by the (unknown) molecule. It will be appreciated that using a GNN trained in accordance with the methods disclosed herein that is able to extract more (useful) information from a graph will lead to improvements in the property prediction task (which involves extracting a representation from a graph indicating a molecule).

In B, a student GNN 10 is used in a link prediction task. It will be appreciated that, depending on what the graph represents, the link prediction may be implemented in, for example, the detection of disease outbreaks, in social network systems for controlling user privacy or detecting spam emails, or in a navigation system to find efficient routes based on current traffic patterns. The student GNN 10 extracts a representation of the graph and then based on the representation (e.g. based on the dense layers) a prediction is made as to whether a particular further link or links exists in the graph. It will be appreciated that using a GNN trained in accordance with the methods disclosed herein that is able to extract more (useful) information from a graph will lead to improvements in the link prediction task (which involves extracting a representation from a graph).

In A and B, the student GNN 10 will have been trained for the specific task, for example during the training in accordance with methods disclosed herein and/or in a separate training step following the training in accordance with methods disclosed herein.

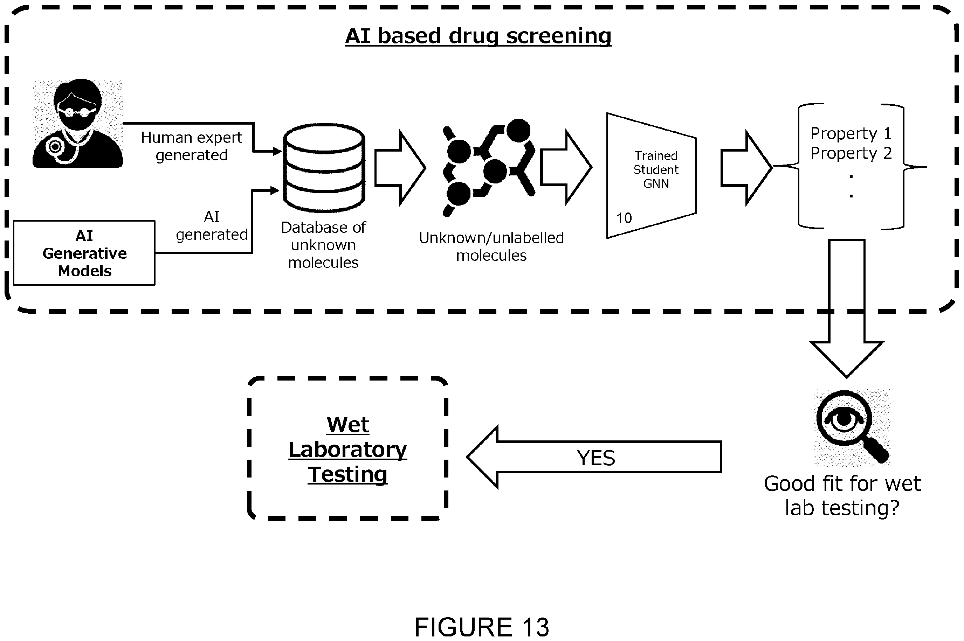

Figure 13 indicates another example implementation of the methods disclosed herein which may be referred to as drug discovery. This may be considered an extension of A in Figure 12. Here, a student GNN 10 trained as disclosed herein is used to predict properties of (unknown) molecules based on graphs indicating the molecules, and then based on these predicted properties it is determined which candidates are selected for wet lab testing. The determination will of course depend on the specific requirements and aims of the method (i.e. what properties are desired). The determination may be considered to be based on a similarity of the (unknown) molecules with a target molecule or target group of molecules, or based on a target property or target group of properties. The unknown molecules may be AI generated or human (expert) generated.

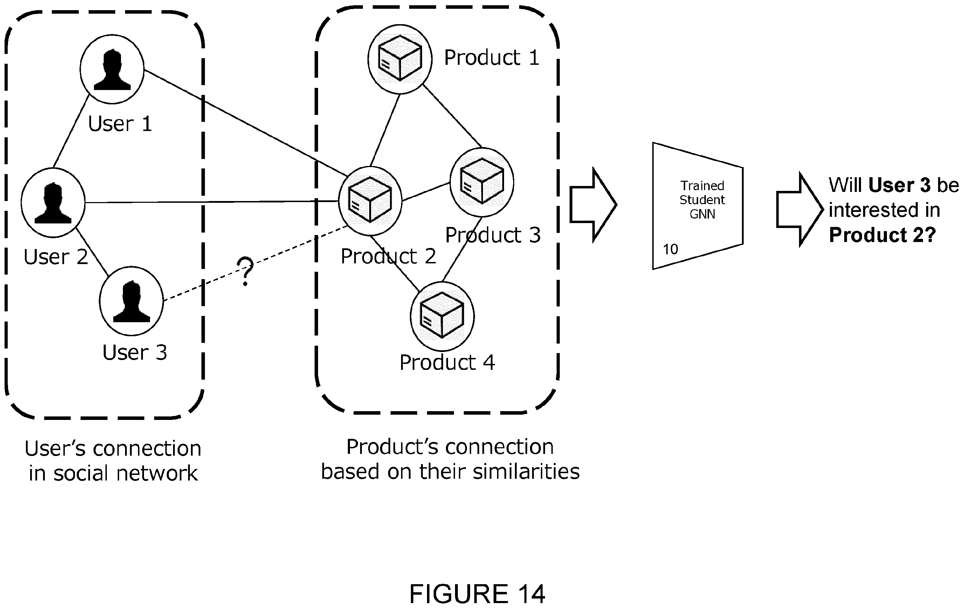

Figure 14 illustrates another example implementation of the methods disclosed herein which may be referred to as a recommendation system. This may be considered an extension/use of B in Figure 12. Here, the graph from which a representation is extracted by the student GNN 10 (trained as disclosed herein) is a graph representing users and products and the users' interest in the products. As shown, the user's connections to each other are based on a social network, and the products' connections to each other are based on similarities between the products. The link predicted is between a node representing a user and a node representing a product, and is used to indicate a likelihood of a user (user 3) being interested in a product (product 2). The prediction may be considered based on the user's similarity to other users and product 2's similarity to other products, and which users are interested in which products. It will be appreciated that the prediction of links may be more accurate if the student GNN 10 is able to extract better representations (i.e. representations with better (semantic and structural) information).

The "task" as used herein may comprise a spam detection task. That is, GNNs trained as disclosed herein may used in a spam detection task. In a spam detection task a graph (input graph) is obtained by considering the available emails/messages as the nodes of the graph. Here the graph edges are created by obtaining the similarity measures among/between various emails/messages. The task is to predict the labels of the unknown nodes. Hence during inference this may be viewed as a node classification task. For example, emails/messages for which a "spam" label is predicted may be flagged/detected. A GNN/model that can extract rich representations of the nodes will be useful for node classification for detecting spam.

The "task" as used herein may comprise a disease outbreak detection task. That is, GNNs trained as disclosed herein may used in a disease outbreak detection task. In disease outbreak detection, cities/localities/areas are considered as the nodes of the input graph and the edges represents a measure of connectivity between the cities/localities/areas. The task is to predict whether a given city will have a disease outbreak (i.e. whether the disease will spread to that city) based on the links and disease spreading in other cities. This task may be viewed as a node classification task.

The "task" as used herein may comprise a route-finding task in which the trained student GNN is used to predict links between nodes in a graph representing e.g. a node network.

Extracting a representation of a graph may be referred to as embedding the graph or generating graph embeddings (or node embeddings) or representation learning. Extracting a representation of a graph may be considered to comprise extracting a representation of each node in the graph, and may be considered comprise combining these node representations (or node-level representations) to obtain a representation of the graph. The node embeddings or node representations may comprise information, for the node, about some or all of the other nodes in the graph and connections thereto (and therebetween).

A GNN may comprise layers which may be referred to as message passing layers, responsible for combining node and edge information into node embeddings. This process may be referred to as graph convolution. For example, a node embedding may comprise information about the node and other nodes (and edges) in its neighborhood. It will be appreciated that there are a number of specific ways in which a representation may be extracted from a graph and the specific method used is not important in the methods disclosed herein. The methods disclosed herein are concerned with training a student GNN as described above and the specific embedding methodology may vary.

As described above, considering conventional methods: prediction-based methods (e.g. comparative method 1) produce representations by learning contextual relationships by predicting local structures such as sub-graphical features including nodes, edges, and subgraphs; Contrastive Learning-based methods (e.g. comparative method 2) produce representations that attract (i.e. make more similar) the representations of an original graph and its perturbed variations, while repelling (i.e. making less similar) representations of the other graph inputs (different graphs); and existing discrepancy-based Self-supervised Learning methods (e.g. comparative method 3) incorporate an edit-distance between the original graphs and its perturbations to introduce the notion of discrepancy in their learned representations.

In summary, some problems with conventional methods are as follows: Comparative Method 1: Does not capture the global structures of graphs. Comparative Method 2: Perturbation in discrete space such as editing edges/nodes can significantly changes graph properties.

Comparative Method 3: Edit distance between an original graph and its perturbed variations cannot capture the accurate semantic difference among different graphs.

The aspects disclosed herein may comprise, given two unlabeled graphs, computing their perceptual distance using the hidden representations of pretrained (teacher) network(s), and using their perceptual distance to train another network (student GNN) along with other loss functions to obtain the final graph representations for downstream tasks.

Aspects disclosed herein are capable of capturing appropriate discrepancy among different variations of perturbed graphs to obtain a better discriminative representation for downstream tasks. Aspects disclosed herein may achieve better performance in various downstream tasks with limited labelled data (such as graph classification, property predictions and link predictions), for example as shown in Figure 11.

Put another way, in conventional methods, hand-picked loss functions are merely proxy for the desired objective for training the existing graph representation learning models. These losses can only enforce a limited semantic information on local graph structures (e.g., predictive learning), often without quantifying the notion of distance (e.g., contrastive learning), or only focusing on structural information with noisy sematic signals (e.g., D-SLA). In some implementation examples disclosed herein, the student GNNs may be considered to be learning not only from the edit distance, but also from other loss functions to capture both semantic and structural differences of different graphs. Disclosed herein is a sample-dependent perceptual loss that captures both semantical and structural differences between graphs from the perspective of the teacher network. It may be considered that the perceptual distance acts as a regularizer for providing a better notion of semantic distances between graphs, as mentioned above. Since aspects disclosed herein may be considered to use a better "ground-truth" for the notion of semantic and structural distance between graphs by incorporating perceptual distance within loss function(s) along with other loss(es) for training, a student GNN can learn a regularized notion of distances between graphs for producing better representations.

An objective of aspects disclosed herein may be considered to be improving performance on downstream tasks (e.g., graph classification, node classification, link prediction) by learning informative representations of graphs with accurate discrepancy using a self-supervised perceptual distance, obtained from teacher network(s), with higher discriminative power, as a proxy for human-intelligence. The proposed training methodologies are generic in that they may be applied to graphs irrespective of their number of nodes or edges or other structural details, or domain/use-case.

Aspects disclosed herein may include a method to learn effective representations for graphs with accurate discrepancy by introducing a self-supervised perceptual loss using a teacher network towards improving on downstream tasks with limited supervisions of labelled data. The teacher network's perception provides the semantic difference between two different graphs.

A graph as disclosed herein may stored (in memory) in the form of linked data nodes.

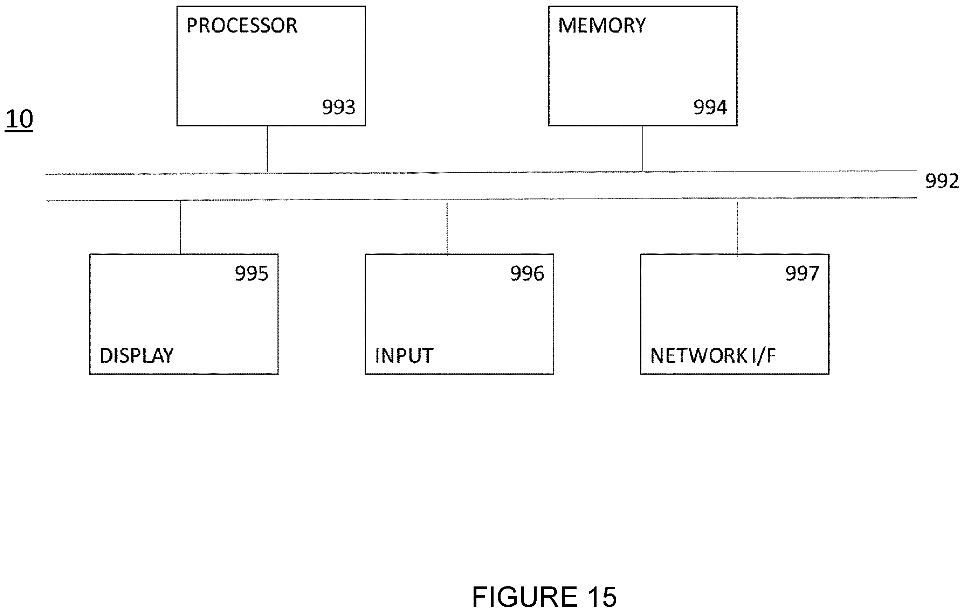

Figure 15 is a block diagram of an information processing apparatus 10 or a computing device 10, such as a data storage server, which embodies the present invention, and which may be used to implement some or all of the operations of a method embodying the present invention, and perform some or all of the tasks of apparatus of an embodiment. The computing device 10 may be used to implement any of the method steps described above, e.g. any of steps S11-S17, S20-S29, S51-S57, S31-S37, and S41-S43.

The computing device 10 comprises a processor 993 and memory 994. Optionally, the computing device also includes a network interface 997 for communication with other such computing devices, for example with other computing devices of invention embodiments. Optionally, the computing device also includes one or more input mechanisms such as keyboard and mouse 996, and a display unit such as one or more monitors 995. These elements may facilitate user interaction. The components are connectable to one another via a bus 992.

The memory 994 may include a computer readable medium, which term may refer to a single medium or multiple media (e.g., a centralized or distributed database and/or associated caches and servers) configured to carry computer-executable instructions. Computer-executable instructions may include, for example, instructions and data accessible by and causing a computer (e.g., one or more processors) to perform one or more functions or operations. For example, the computer-executable instructions may include those instructions for implementing a method disclosed herein, or any method steps disclosed herein, for example any of steps S11-S17, S20-S29, S51-S57, S31-S37, and S41-S43. Thus, the term "computer-readable storage medium" may also include any medium that is capable of storing, encoding or carrying a set of instructions for execution by the machine and that cause the machine to perform any one or more of the method steps of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing computer program code stored in the memory 994 to implement any of the method steps described herein. The memory 994 stores data being read and written by the processor 993 and may store GNNs and/or graphs and/or images and/or weights and/or error amounts and/or target disagreements and/or other data, described above, and/or programs for executing any of the method steps described above. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and operations discussed herein. The processor 993 may be considered to comprise any of the modules described above. Any operations described as being implemented by a module may be implemented as a method by a computer and e.g. by the processor 993.

The display unit 995 may display a representation of data stored by the computing device, such as a representation of graphs and/or predictions based thereon and/or GUI windows and/or interactive representations enabling a user to interact with the apparatus 10 by e.g. drag and drop or selection interaction, and/or any other output described above, and may also display a cursor and dialog boxes and screens enabling interaction between a user and the programs and data stored on the computing device. The input mechanisms 996 may enable a user to input data and instructions to the computing device, such as enabling a user to input any user input described above.

The network interface (network I/F) 997 may be connected to a network, such as the Internet, and is connectable to other such computing devices via the network. The network I/F 997 may control data input/output from/to other apparatus via the network. Other peripheral devices such as microphone, speakers, printer, power supply unit, fan, case, scanner, trackerball etc may be included in the computing device.

Methods embodying the present invention may be carried out on a computing device/apparatus 10 such as that illustrated in Figure 15. Such a computing device need not have every component illustrated in Figure 15, and may be composed of a subset of those components. For example, the apparatus 10 may comprise the processor 993 and the memory 994 connected to the processor 993. Or the apparatus 10 may comprise the processor 993, the memory 994 connected to the processor 993, and the display 995. A method embodying the present invention may be carried out by a single computing device in communication with one or more data storage servers via a network. The computing device may be a data storage itself storing at least a portion of the data.

A method embodying the present invention may be carried out by a plurality of computing devices operating in cooperation with one another. One or more of the plurality of computing devices may be a data storage server storing at least a portion of the data.

The invention may be implemented in digital electronic circuitry, or in computer hardware, firmware, software, or in combinations of them. The invention may be implemented as a computer program or computer program product, i.e., a computer program tangibly embodied in a non-transitory information carrier, e.g., in a machine-readable storage device, or in a propagated signal, for execution by, or to control the operation of, one or more hardware modules.

A computer program may be in the form of a stand-alone program, a computer program portion or more than one computer program and may be written in any form of programming language, including compiled or interpreted languages, and it may be deployed in any form, including as a stand-alone program or as a module, component, subroutine, or other unit suitable for use in a data processing environment. A computer program may be deployed to be executed on one module or on multiple modules at one site or distributed across multiple sites and interconnected by a communication network.

Method steps of the invention may be performed by one or more programmable processors executing a computer program to perform functions of the invention by operating on input data and generating output. Apparatus of the invention may be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

The above-described embodiments of the present invention may advantageously be used independently of any other of the embodiments or in any feasible combination with one or more others of the embodiments.

## Claims

1. A computer-implemented method comprising performing a training process, the training process comprising:
using a student graph neural network, GNN, to extract a pair of first representations from a pair of training graphs, respectively;
computing a disagreement between the first representations;
using at least one teacher GNN to extract a pair of second representations from the pair of training graphs, respectively;
computing a perceptual distance between the second representations;
comparing the disagreement between the first representations with a target disagreement which is based at least in part on the perceptual distance between the second representations; and
adjusting at least one weight of the student GNN based on the comparison.

2. The computer-implemented method as claimed in claim 1, wherein the training process comprises:
using each of a plurality of teacher GNNs to extract a pair of second representations from the pair of training graphs, respectively;
for each pair of second representations, computing a perceptual distance between the second representations of the pair; and
computing an average of the plurality of perceptual distances,
wherein the target disagreement is based at least in part on the average of the plurality of perceptual distances.

3. The computer-implemented method as claimed in claim 1 or claim 2, wherein the training process further comprises, before using the at least one teacher GNN to extract the pair of second representations, training the at least one teacher GNN.

4. The computer-implemented method as claimed in any of claims 1-3, wherein computing the perceptual distance between the second representations comprises computing a Euclidean distance between the second representations.

5. The computer-implemented method as claimed in any of the preceding claims, wherein the target disagreement is based in part on a difference between the pair of training graphs concerned.

6. The computer-implemented method as claimed in any of the preceding claims, wherein:
the target disagreement includes a task-based term based on a task-based target disagreement between the first representations in accordance with a task; and/or
the training process further comprises comparing at least one of the first representations with a target representation which is based on a task, and adjusting at least one weight of the student GNN based on the comparison.

7. The computer-implemented method as claimed in any of the preceding claims, comprising, after the training process, using the student graph to perform a task.

8. The computer-implemented method as claimed in claim 6 or claim 7, wherein the task comprises any of:
extracting graph representations from graphs which represent molecules;
extracting graph representations from graphs representing interactions between users and products;
extracting graph representations from graphs representing locations of cases of a disease;
extracting graph representations from graphs representing traffic patterns; and
extracting graph representations from graphs representing emails and messages.

9. The computer-implemented method as claimed in any of the preceding claims, wherein
the pair is a first pair comprising a reference training graph and an augmented version of the reference training graph;
the training process further comprises performing the first and second representations extraction and the disagreement and perceptual distance computation steps based on a second pair of training graphs, the second pair of training graphs comprising the reference graph and another reference graph;
comparing the disagreement with the target disagreement comprises comparing a first difference between the disagreements corresponding to the first and second pairs with a second difference between the perceptual distances corresponding to the first and second pairs; and
adjusting the at least one weight comprises adjusting the at least one weight based on the comparison between the first and second differences.

10. The computer-implemented method as claimed in claim 9, wherein the adjustment is to bring the first difference at least above the second difference.

11. The computer-implemented method as claimed in claim 9, wherein if the second difference is below a user-defined threshold, the adjustment is to bring the first difference at least above the user-defined threshold.

12. The computer-implemented method as claimed in claim 6 or claim 7, wherein the task comprises any of:
predicting a link in a graph representing users and products and interactions therebetween to predict whether a user will be interested in a product;
classifying a graph representing a molecule;
classifying a node in a graph in which nodes represent emails and/or messages and edges represent the similarity between the emails and/or messages, the classification comprising a classification as a spam email or not a spam email;
classifying a node in a graph in which nodes represent geographical areas and links represent a measure of connectivity between the geographical areas concerned, the classification comprising a classification as a geographical area having a disease outbreak or a geographical area not having a disease outbreak.

13. The computer-implemented method as claimed in any of the preceding claims, wherein using the student GNN to extract the pair of first representations from the pair of training graphs, respectively, comprises using two instances of the student GNN with shared parameters.

14. A computer program which, when run on a computer, causes the computer to carry out a method comprising performing a training process, the training process comprising:
using a student graph neural network, GNN, to extract a pair of first representations from a pair of training graphs, respectively;
computing a disagreement between the first representations;
using at least one teacher GNN to extract a pair of second representations from the pair of training graphs, respectively;
computing a perceptual distance between the second representations;
comparing the disagreement between the first representations with a target disagreement which is based at least in part on the perceptual distance between the second representations; and
adjusting at least one weight of the student GNN based on the comparison

15. An information processing apparatus comprising a memory and a processor connected to the memory, wherein the processor is configured to perform a training process, the training process comprising:
using a student graph neural network, GNN, to extract a pair of first representations from a pair of training graphs, respectively;
computing a disagreement between the first representations;
using at least one teacher GNN to extract a pair of second representations from the pair of training graphs, respectively;
computing a perceptual distance between the second representations;
comparing the disagreement between the first representations with a target disagreement which is based at least in part on the perceptual distance between the second representations; and
adjusting at least one weight of the student GNN based on the comparison.
